Europäisches Patentamt

European Patent Office

Office européen des brevets

(19)

(11) Veröffentlichungsnummer: **0 247 559**
**B1**

(12) # EUROPÄISCHE PATENTSCHRIFT

(45) Veröffentlichungstag der Patentschrift:
10.10.90

(51) Int. Cl.⁵: **A61M 25/01**

(21) Anmeldenummer: **87107583.4**

(22) Anmeldetag: **25.05.87**

(54) **Katheter mit Einführungshilfe.**

(30) Priorität: 26.05.86 CH 2116/86
07.08.86 DE 3626711

(43) Veröffentlichungstag der Anmeldung:
02.12.87 Patentblatt 87/49

(45) Bekanntmachung des Hinweises auf die Patenterteilung:
10.10.90 Patentblatt 90/41

(84) Benannte Vertragsstaaten:
AT BE CH DE ES FR GB GR IT LI LU NL SE

(56) Entgegenhaltungen:
DE-A- 2 823 025
US-A- 4 062 363

(73) Patentinhaber: Sterimed Gesellschaft für medizinischen Bedarf mbH, Fasanerieweg 15-17 Postfach 215,
D-6600 Saarbrücken 3(DE)

(72) Erfinder: Kramann, Bernhard, Preussenstrasse 43,
D-6650 Homburg/Saar(DE)

(74) Vertreter: RUPP, Herbert Byk Gulden Lomberg Chemische Fabrik GmbH et al,
Byk-Gulden-Strasse 2 Postfach 6500,
D-7750 Konstanz(DE)

## Beschreibung

### Technisches Gebiet

Die Erfindung bezieht sich auf einen Katheter mit Einführungshilfe nach dem Prinzip des sich unter Einwirkung hydraulischen oder pneumatischen Drucks ausstülpenden Schlauchs.

### Stand der Technik

Beim Einführen von Kathetern in Öffnungen des menschlichen Körpers ist es im allgemeinen wünschenswert, den Kontakt des Katheters mit der Wand des Körperkanals möglichst schonend zu gestalten oder ganz zu vermeiden. Es ist seit langem bekannt, daß z.B. beim Einführen von Kathetern in die Harnblase Keime aus der Harnröhre in die Harnblase verschleppt werden [H.F.Helmholz Sr., The Journal of Urology, 64(1950)158-166]. Diese transurethrale Keimverschleppung kann zu · Infektionen der Harnblase führen. Bei Untersuchungen von Urin, der durch konventionelle Katheterisierung oder nach der Methode des Mittelstrahlurins gewonnen wird, besteht eine erhebliche Gefahr von falsch positiven Urinbefunden mit der nachfolgenden unnötigen Behandlung mit antibiotischen Substanzen.

In der US-A 4043345 wird ein Katheter beschrieben, bei dem der Katheterschlauch in ein starres Röhrchen eingestülpt ist, das am patientenfernen Ende des Katheterschlauchs angebracht ist. Durch Beaufschlagen ·des Röhrchens mit hydraulischem Druck, z.B. aus einer Spritze, kann der Katheterschlauch aus dem Röhrchen herausgestülpt werden. Setzt man das Röhrchen zu Beginn des Ausstülpens an der Mündung der Harnröhre an, so stülpt sich der Katheterschlauch ohne Gleitbewegung gegenüber der Wand der Harnröhre in die Harnröhre hinein. Nach Beendigung des Ausstülpvorganges öffnet sich ein Ventil am patientennahen Ende des Katheterschlauchs. Trotz der überzeugenden Funktionsweise verschwand dieser Katheter wieder vom Markt,· weil keine ökonomische Möglichkeit der Massenherstellung gefunden wurde.

In der US-A 3502069 wird eine Vorrichtung zum Einführen von medizinischen Instrumenten, wie z.B. Kathetern, in Körperöffnungen beschrieben. Am patientennahen Ende eines rohrförmigen Gehäuses ist eine dünne schlaucharti ge Hülle befestigt. Diese Hülle ist in das Gehäuse eingestülpt und am anderen Ende durch eine Verschlußvorrichtung verschlossen. Durch Überdruck kann die Hülle in Körperöffnungen eingestülpt werden. Die Verschlußvor- richtung kann entfernt werden, sobald die Hülle ausgestülpt ist. Es ist dann möglich z.B. einen Katheter durch die Hülle in die Körperöffnung zu schieben. Als Verschlußvorrichtung wird z.B. eine Federklemme vorgeschla- gen, die nach dem Ausstülpen der Hülle über einen Faden oder einen dünnen Stab abgezogen werden kann. Die Anwendung dieser Vorrichtung für eine Harnblasenkatheterung ist umständlich: Nach Einstülpen der dünnen Hülle muß die Verschlußvorrichtung entfernt werden, bevor der Katheter eingeführt wird. In einer anderen Ausführungsform wird vorgeschlagen, das offene Ende der Hülle zu verkleben.

Weitere Vorrichtungen, welche nach dem Prinzip des sich unter Einwirkung von hydraulischem Druck ausstülpenden Schlauches arbeiten, sind aus US-A 3669099, US-A 3433215 und US-A 3168092 bekannt.

Kathetervorrichtungen, die nach dem Prinzip des ausstülpbaren Schlauchs arbeiten, haben sich für Wegwerfartikel als zu kostspielig erwiesen. Wegen der erläuterten Nachteile konnte sich bislang keine dieser Kathetervorrichtungen in der Praxis durchsetzen.

### Beschreibung der Erfindung

Aufgabe der vorliegenden Erfindung ist es, einen Katheter mit einem sich unter Einwirkung hydraulischen oder pneumatischen Drucks ausstülpbaren Schlauch als Einführungshilfe zur Verfügung zu stellen, der konstruktiv und in der Anwendung einfacher ist, als diejenigen nach dem Stand der Technik.

Gelöst wird die Aufgabe dadurch, daß der Katheter sich vollständig in einem am patientenfernen Ende verschlossenen dünnwandigen flexiblen Schlauchgebilde befindet, das von seinem patientennahen Ende her teilweise nach innen in den Zwischenraum zwischen Katheter und seinem nicht eingestülpten Teil eingestülpt ist.

Gegenstand der Erfindung sind die in den Ansprüchen und der Beschreibung definierten Gegenstände.

Die Gestaltung des Katheters ist von der gewünschten Anwendung abhängig. Geeignet sind sowohl starre als auch flexible Katheter, wie sie für die gewünschte Anwendung üblicherweise verwendet werden. Bevorzugt werden flexible Katheter aus den üblichen für Katheter verwendeten Materialien. Der Katheter ist an seinem patientenfernen Ende auf eine hydraulischen oder pneumatischen Druck erzeugende Vorrichtung aufsetzbar gestaltet. Als hydraulischen oder pneumatischen Druck erzeugende Vorrichtung kommt vorzugsweise eine übliche Injektionsspritze in Frage.

Als dünnwandiges flexibles Schlauchgebilde kommen Folienschläuche aus für Folien üblichen Materialien in Betracht. Der Durchmesser des Folienschlauches wird so bemessen, daß er locker über den Katheter gezogen werden kann. Er soll den Katheter so locker umschließen, daß man ihn auf dem Katheter ziehharmonikaartig zusammenschieben kann. Der Folienschlauch ist ungefähr so lang, daß er den Katheter aufnehmen kann und daß das patientennahe Ende um ungefähr die Länge des auszukleidenden Körperkanals eingestülpt werden kann. Das patientennahe Ende des Schlauchgebildes ist entweder offen oder geschlossen, wobei sich der Verschluß nach dem Ausstülpen selbsttätig öffnet. Es kann z.B. durch eine Sollbruchstelle verschlossen werden, die nach dem Ausstülpen durch den angewendeten Überdruck aufreißt. Diese Sollbruchstelle kann z.B. durch eine bandförmige Quetschung im Bereich des Endes des eingestülpten Teils erzeugt werden. Es hat sich gezeigt, daß die Wände des eingestülpten Teils des Schlauchgebildes durch Adhäsionskräfte genügend aneinander anliegen um eine ausreichende Abdichtung zu erzeugen. Das Ende des eingestülpten Teils des Schlauchgebildes kann deshalb auch offenbleiben. Bei Ausstülpung mittels Drucks wird der eingestülpte Teil des Schlauchgebildes zusätzlich zusammengepreßt, wodurch eine zusätzliche Abdichtung erreicht wird.

Ein an die Verwendung zur Katheterung der Harnblase von Frauen angepaßter flexibler Katheter aus PVC ist beispielsweise etwa 12 bis 20 cm, vorzugsweise etwa 16 cm lang und weist einen Durchmesser von 3 bis 5 mm, vorzugsweise von etwa 4 mm auf. Der Katheterschlauch ist am patientennahen Ende offen oder weist seitliche Öffnungen (Katheteraugen) auf. Das patientenferne Ende ist in ein kurzes Schlauchanschlußstück oder hülsenförmiges Formteil eingeklebt, um das patientenferne Ende mit dem Anschlußstutzen einer Injektionsspritze verbinden zu können. Der Katheter befindet sich in einem Polyethylenschlauch von 18 bis 26 cm, vorzugsweise etwa 22 cm Länge, der in flachem Zustand eine Breite von 4 bis 14 mm, vorzugsweise etwa 9 mm aufweist. Die Stärke der Folie beträgt etwa 0,01 bis 0,04, vorzugsweise etwa 0,02 mm. Ein Ende des Polyethylenschlauches ist verschweißt. Der Katheter befindet sich im Polyethylenschlauch, wobei sein patientenfernes Ende in der Nähe der Schweißnaht zu liegen kommt. Das überstehende Ende des Polyethylenschlauchs ist in den Teil des Polyethylenschlauchs eingestülpt, in dem der Katheter liegt. Die Länge des eingestülpten Endes beträgt etwa 4 bis 7 cm, vorzugsweise etwa 5 bis 6 cm.

Die Dimensionierung des erfindungsgemäßen Katheters für andere Verwendungszwecke, wie z.B. als Männerkatheter, liegt für den Fachmann auf der Hand.

Um das Ansetzen des Katheters an einer Körperöffnung, wie z.B. der Harnröhre, und das Einstülpen des Schlauchgebildes in die Körperöffnung zu erleichtern, kann eine das patientennahe Ende des Katheters samt Schlauchgebilde umschließende Führungshülse vorgesehen werden. Der Innendurchmesser der Führungshülse ist so bemessen, daß der Katheter samt Schaluchgebilde ohne beeinträchtigende Reibung durch die Führungshülse geschoben werden kann. Die Führungshülse kann am patientenfernen Ende gewünschtenfalls mit Vorrichtungen zur besseren Handhabung, wie z.B. Griffplatten ausgestattet sein. Zweckmäßigerweise kann sich der Innendurchmesser der Führungshülse nach patientenfern konisch erweitern.

Die Anwendung einer erfindungsgemäßen Kathetervorrichtung erfolgt, indem das patientenferne Ende des Katheters auf den Anschlußstutzen einer Injektionsspritze aufgesetzt wird, wobei das dünnwandige flexible Schlauchgebilde durchstoßen wird. Beim Durchstoßen wird der im Bereich der Durchstoßstelle liegende Teil des Schlauchgebildes zwischen den Anschlußstutzen der Injektionsspritze und das patientenferne Ende des Katheters eingeklemmt. Dadurch bleibt das Innere des Schaluchgebildes gegenüber der Umgebung abgedichtet. Das patientennahe Ende des Katheters wird an den Eingang der zu katheterisierenden Körperöffnung angelegt. Sobald durch die aufgesetzte Spritze hydraulischer oder pneumatischer Druck erzeugt wird, stülpt sich der eingestülpte Teil des Schlauchgebildes aus und dringt in die Körperöffnung ein. Hierbei wird die Wand des Körperkanals durch das Schlauchgebilde ausgekleidet. Sobald die Ausstülpung beendet ist, entweicht ein Teil des Druckmediums in die Körperhöhle. Falls das patientennahe Ende des Schlauchgebildes durch eine Sollbruchstelle verschlossen war, platzt diese auf. Der Katheter kann nun in der durch das Schlauchgebilde ausgekleideten Höhle vorgeschoben werden. Gewünschtenfalls kann die Injektionsspritze vor dem Vorschieben des Katheters abgenommen werden. Es ist zweckmäßig, das Schlauchgebilde festzuhalten, um dessen Verschiebung in dem Körperkanal zu vermeiden. Beim Vorschieben des Katheters faltet sich der patientenferne Teil des Schlauchs zwischen Ende und der Stelle, an der festgehalten wird, ziehharmonikaartig auf. Bei absatzweisem Vorschieben des Katheters kann die der jeweiligen Vorschubstrecke des Katheters entsprechende Länge des Schlauchgebildes nach patientenfern vom Katheter abgestreift werden. Sobald der Katheter seine Endlage erreicht hat, kann über die angesetzte Injektionsspritze oder nach Abnahme der Injektionsspritze direkt Flüssigkeit aus der Körperhöhle entnommen werden oder über eine zweite Injektionsspritze Flüssigkeit zugeführt werden. Gewünschtenfalls kann das Schlauchgebilde auch nach patientenfern vom Katheter abgezogen werden. Dieses Abziehen kann absatzweise jeweils um etwas weniger als die Distanz, die der Katheter mit seinem patientenfernen Ende aus der Körperöffnung herausragt, erfolgen. Wenn das Schlauchgebilde nicht nach patientenfern abgezogen wird, ist es für den Fall, daß aus der Körperhöhle Flüssigkeit abgeführt werden soll, selbstverständlich angezeigt, den Katheter so weit durch das Schlauchgebilde in die Körperöffnung einzuführen, daß die patientennahe Öffnung bzw. die seitlichen Öffnungen (Augen) des Katheters außerhalb des patientennahen Endes des Schlauchgebildes zu liegen kommen. Katheter und/oder Schlauchgebilde können gewünschtenfalls entsprechende Markierungen aufweisen, die dem Untersucher die Bestimmung der korrekten Lage des Katheters erleichtern.

Gewünschtenfalls kann die erfindungsgemäße Kathetervorrichtung auch mit einer Führungshülse ausgestattet sein. In diesem Fall wird das patientennahe Ende der Führungshülse an die zu katheterisierende Körperöffnung angelegt und das Schlauchgebilde wie vorstehend beschrieben in die Körperöffnung eingestülpt. Die Führungshülse kann gewünschtenfalls nach Abnehmen der Injektionsspritze in Richtung Untersucher entfernt werden. Bei Verwendung einer in Längsrichtung teilbaren Führungshülse kann diese bereits vor Abnahme der Injektionsspritze entfernt werden.

Gegenüber den Kathetern mit Einführungshilfe nach dem Stand der Technik ist die erfindungsgemäße Kathetervorrichtung sehr kostengünstig in der Herstellung und ohne besonderen Aufwand anwendbar. Gegenüber den Kathetern, bei denen der Katheterschlauch selbst ausgestülpt wird, hat der Untersucher wegen des viel geringeren Drucks, der zum Ausstülpen des Schlauches erforderlich ist, ein viel feineres Gefühl für etwaige Verengungen in dem Körperkanal. Durch den sehr viel niedrigeren Arbeitsdruck verringert sich die Verletzungsgefahr für den Patienten erheblich. Die erfindungsgemäße Vorrichtung hat darüber hinaus den Vorteil einer guten Sterilisierbarkeit.

Die Herstellung des Erfindungsgegenstandes kann beispielsweise in folgenden Schritten erfolgen: Ein Folienschlauch wird einseitig verschlossen und durch Einblasen von Luft leicht aufgebläht. Nachdem auch das andere Ende verschlossen ist, wird der Schlauch von einem Ende her z.B. mittels eines Rundstabes passenden Durchmessers eingestülpt. Nachdem das Ende genügend weit eingestülpt ist, wird der Rundstab entfernt. Sodann werden die beiden geschlossenen Enden abgeschnitten und das im Schlauch liegende Ende ein Stück aus dem außenliegenden Teil des Schlauches herausgezogen und mit einer Sollbruchstelle verschlossen. Der Verschluß des Endes des innenliegenden Teils des Schlauchs mit einer Sollbruchstelle kann beispielsweise durch Quetschen oder Perforieren erfolgen. Die Festigkeit der Sollbruchstelle ist zweckmäßigerweise nur so groß, daß während des Ausstülpvorganges ein Entweichen des fluiden Druckmediums verhindert wird und nach Beendigung des Ausstülpvorgangs ein Aufgehen gewährleistet ist. Nach Anbringen der Sollbruchstelle wird das außenliegende Ende des Schlauches über das innenliegende Ende gezogen. Nachdem ein Katheter mit dem patientennahen Ende voraus durch das offene Ende des Schlauchs eingeführt wurde, wird das offene Ende des Schlauchs fest verschlossen, beispielsweise durch Verschweißen. Gewünschtenfalls kann das Ende des Schlauchs auch mit dem patientenfernen Ende des Katheters fest verbunden werden, so daß das Ende des Katheters direkt von außen zugänglich ist. Das Verbinden kann beispielsweise durch Verkleben oder Verschweißen erfolgen. Bei dieser Ausführungsform wird bei der Anwendung der Vorrichtung das Schlauchgebilde nicht nach patientenfern vom Katheter abgezogen, sondern verbleibt in ziehharmonikaartig zusammengeschobener Form auf dem patientenfernen Teil des Katheters.

Nachstehend wird die Erfindung anhand der schematischen Querschnitte der Figuren 1 bis 5 erläutert. In allen Figuren haben die Bezugszeichen die gleiche Bedeutung.

Fig. 1 zeigt einen Katheter in einem Schlauchgebilde;
Fig. 2 zeigt einen Katheter in einem Schlauchgebilde gemäß der Schnittlinie A-A in Fig. 1;
Fig. 2a zeigt den patientennahen Teil eines Katheters nach Fig. 2a mit einer Führungshülse;
Fig. 3 zeigt einen vergrößerten Querschnitt eines Katheters gemäß der Schnittlinie B-B in Fig. 1;
Fig. 4 zeigt einen Katheter in einem Schlauchgebilde mit einer an den Katheter angesetzten Injektionsspritze;
Fig. 4a zeigt eine Detailvergrößerung gemäß dem Ausschnitt C in Fig. 4;
Fig. 5 zeigt einen Katheter in einem teilweise ausgestülpten Schaluchgebilde;
Fig. 5a zeigt einen Katheter in einem teilweise ausgestülpten Schaluchgebilde mit einer Führungshülse;
Fig. 6 zeigt einen Katheter nach Einführung durch das Schlauchgebilde.

In allen Figuren sind die Abstände zwischen Katheter 6 und Schlauchgebilde 1 der besseren Übersichtlichkeit wegen vergrößert dargestellt.

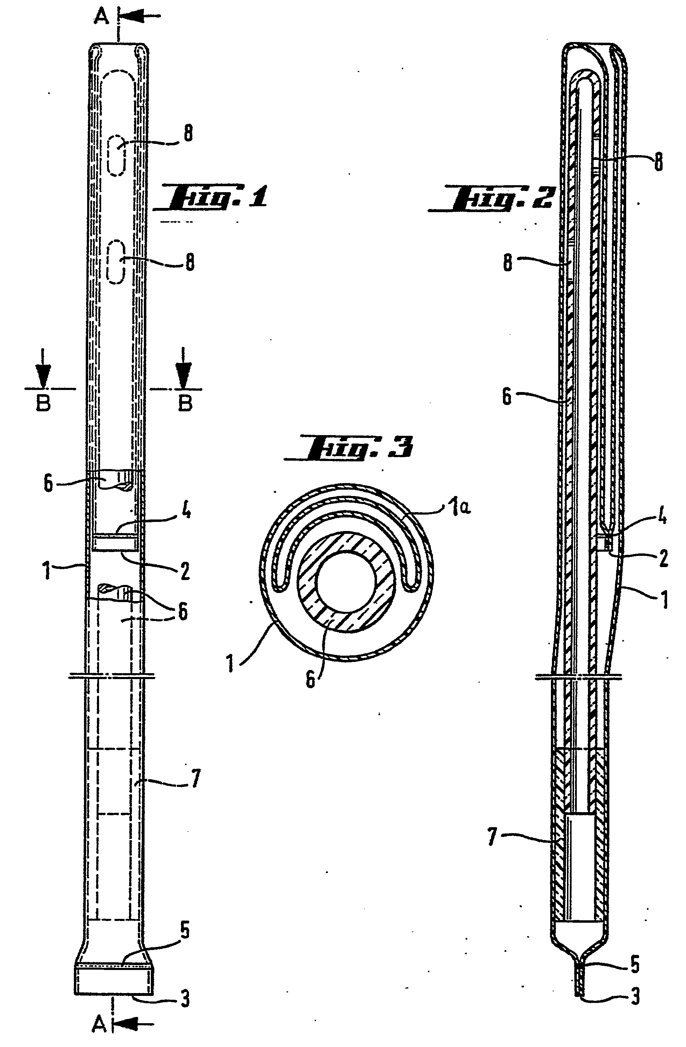

In Fig. 1 ist ein Schlauchgebilde 1 dargestellt, dessen patientennahes Ende 2 eingestülpt ist. Im Bereich dieses Endes 2 ist eine Sollbruchstelle 4 angedeutet. Nahe dem patientfernen Ende 3 ist das Schlauchgebilde 1 durch eine Verschweißung 5 geschlossen.

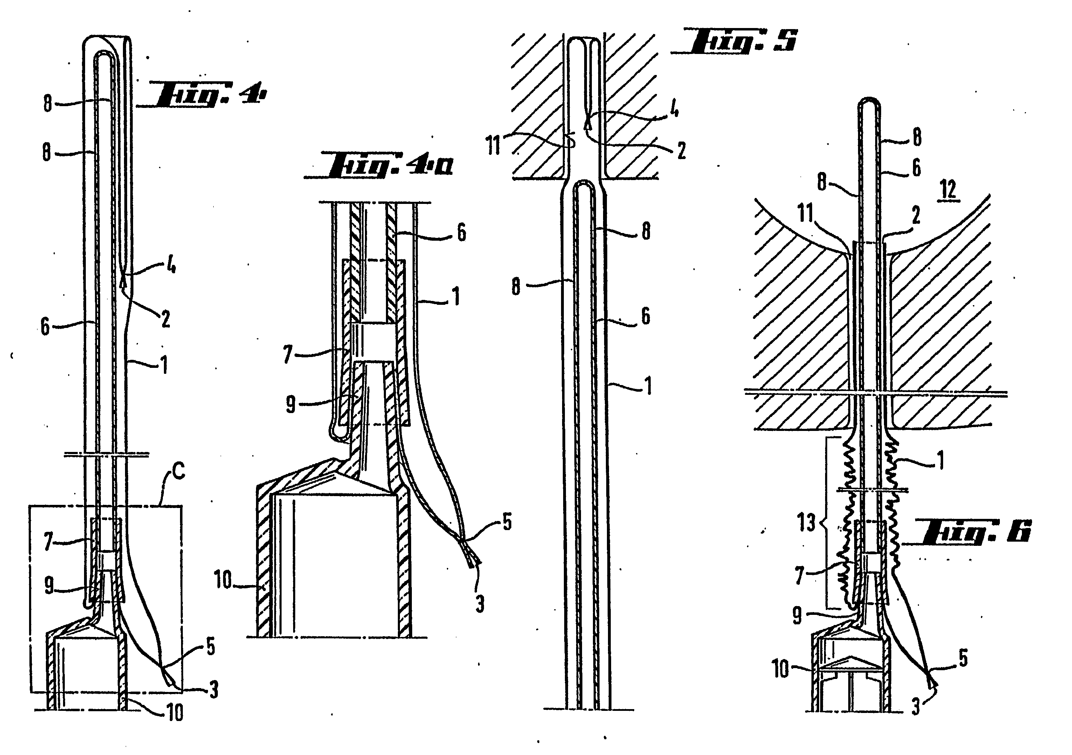

Fig. 2 zeigt einen Querschnitt entlang der Linie A-A in Fig. 1 durch ein Schlauchgebilde mit eingelegtem Katheter 6. Katheter 6 weist am patientenfernen Ende ein Anschlußstück 7 und im Bereich des patientennahen Endes seitliche Katheteraugen 8 auf.

Nach Fig. 2a ist auf den patientennahen Teil eines Katheters 6 samt Schlauchgebilde 1 nach Fig. 2 eine Führungshülse 14 aufgezogen. Diese Führungshülse 14 ist patientennah zylindrisch und erweitert sich nach patientenfern konisch. Das patientennahe Ende 2 des Schlauchgebildes 1 ist nach der Ausführungsform der Fig. 2a nicht durch einen besonderen Verschluß verschlossen. Die Abdichtung des Innenraums des Schlauchgebildes 1 gegenüber der Atmosphäre wird durch die Adhäsionskräfte der aneinanderliegenden Oberflächen des eingestülpten Teils des Schlauchgebildes bewirkt.

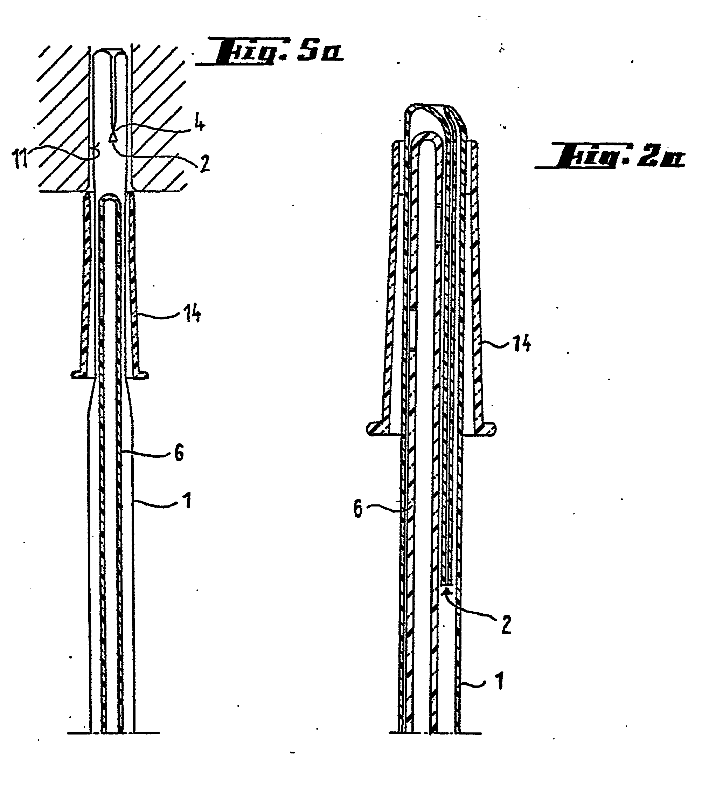

Aus Fig. 3, die einen Querschnitt entlang der Linie B-B in Fig. 1 zeigt, ist ersichtlich, wie Katheter 6 in dem Schlauchgebilde 1 neben dem eingestülpten Teil 1a des Schlauchgebildes 1 liegt.

Fig. 4 zeigt die Vorrichtung von Fig. 2, wobei das Anschlußstück 7 des Katheters 6 auf den Anschlußstutzen 9 einer nur teilweise dargestellten Injektionsspritze 10 unter Perforierung des Schlauchgebildes 1 aufgesetzt ist.

Aus der Detailzeichnung Fig. 4a ist zu entnehmen, wie der Anschlußstutzen 9 einer Injektionsspritze das Schlauchgebilde 1 durchdrungen hat und in das Anschlußstück 7 des Katheters 6 eingesteckt ist, wobei die Ränder der beim Durchstoßen des Schlauchgebildes 1 entstandenen Loches eingeklemmt werden.

In Fig. 5 ist dargestellt, wie das Schlauchgebilde 1 teilweise ausgestülpt und in einen Körperkanal 11 eingestülpt ist.

Fig. 5a unterscheidet sich von Fig. 5 nur dadurch, daß der patientennahe Teil des Katheters 6 samt dem Schlauchgebilde 1 von einer Führungshülse 14 umschlossen wird. Die Führungshülse 14 ist an der Öffnung des Körperkanals 11 angesetzt.

In Fig. 6 hat das patientennahe Ende des Katheters 6 die Körperhöhle 12 erreicht. Der patientenferne Teil des Schlauchgebildes 1 ist im patientenfernen Bereich 13 des Katheters 6 durch das Zusammenschieben beim Vorschieben des Katheters 6 ziehharmonikaartig aufgefaltet.

Die in den nachstehenden Patentansprüchen verwendeten Bezugszeichen sollen lediglich dem Verständnis dienen und keinesfalls als den Schutzumfang beschränkend ausgelegt werden.

Patentansprüche

1. Katheter mit einem sich unter Einwirkung hydraulischen oder pneumatischen Drucks ausstülpenden Schlauch als Einführungshilfe, dadurch gekennzeichnet, daß der Katheter (6) sich vollständig in einem am patientenfernen Ende verschlossenen dünnwandigen flexiblen Schlauchgebilde (1) befindet, das von seinem patientennahen Ende (2) her teilweise nach innen in den Zwischenraum zwischen Katheter (1) und seinem nicht eingestülpten Teil eingestülpt ist.

2. Vorrichtung nach Anspruch 1, dadurch gekennzeichnet, daß das Schlauchgebilde (1) im Bereich des patientennahen Endes (2) durch eine Sollbruchstelle (4) verschlossen ist.

3. Vorrichtung nach Anspruch 1, dadurch gekennzeichnet, daß der Katheter (6) an seinem patientenfernen Ende ein Anschlußstück zum Verbinden mit dem Anschlußstutzen einer Injektionsspritze aufweist.

4. Vorrichtung nach Anspruch 1, dadurch gekennzeichnet, daß das patientenferne Ende (3) des Schlauchgebildes (1) fest mit dem patientenfernen Ende des Katheters (6) verbunden ist.

5. Vorrichtung nach Anspruch 1, dadurch gekennzeichnet, daß zusätzlich eine den patientennahen Teil des Katheters (6) samt Schlauchgebilde (1) umschließende Führungshülse (14) vorgesehen ist.

6. Vorrichtung nach Anspruch 1, dadurch gekennzeichnet, daß sie für die Verwendung als Harnblasenkatheter für Frauen dimensioniert ist.

7. Vorrichtung nach Anspruch 6, dadurch gekennzeichnet, daß der Katheter (6) ungefähr 16 cm lang ist und einen Durchmesser von ungefähr 4 mm aufweist.

8. Vorrichtung nach Anspruch 6, dadurch gekennzeichnet, daß das Schlauchgebilde (1) etwa 22 cm lang ist und in flachem Zustand eine Breite von etwa 9 mm aufweist.

9. Vorrichtung nach Anspruch 6, dadurch gekennzeichnet, daß die Länge des einstülpten Teils des Schlauchgebilde (1) ungefähr 5 bis 6 cm beträgt.

**Claims**

1. Catheter with a tube turning out under the effect of hydraulic or pneumatic pressure and serving as an insertion aid, characterized in that the catheter (6) is situated completely in a thin-walled, flexible tube structure (1) sealed at the end remote from the patient, which tube structure, from its end (2) facing the patient, is turned partially inwards into the intermediate space between the catheter (6) and its part which is not turned in.

2. Device according to Claim 1, characterized in that the tube structure (1) is sealed in the area of the end (2) facing the patient by means of a predetermined breaking point (4).

3. Device according to Claim 1, characterized in that the catheter (6) has, at its end remote from the patient, an attachment piece for connection to the attachment branch of an injection syringe.

4. Device according to Claim 1, characterized in that the end (3) of the tube structure (1) remote from the patient is connected securely to that end of the catheter (6) remote from the patient.

5. Device according to Claim 1, characterized in that a guide sleeve (14) is additionally provided, which surrounds that part of the catheter (6) and tube structure (1) facing the patient.

6. Device according to Claim 1, characterized in that it is dimensioned for use as a urinary bladder catheter for women.

7. Device according to Claim 6, characterized in that the catheter (6) is approximately 16 cm long and has a diameter of approximately 4 mm.

8. Device according to Claim 6, characterized in that the tube structure (1) is about 22 cm long and has a width of about 9 mm in the flat state.

9. Device according to Claim 6, characterized in that the length of the turned-in part of the tube structure (1) amounts to approximately 5 to 6 cm.

**Revendications**

1. Cathéter comportant comme auxiliaire d'introduction un tuyau souple s'élargissant sous l'effet d'une pression hydraulique ou pneumatique, caractérisé en ce que le cathéter (6) est entièrement situé dans un élément tubulaire (1) flexible, à paroi mince, fermé à l'extrémité éloignée du patient, et qui, à partir de son extrémité (2) située du côté du patient, est partiellement replié vers l'intérieur dans l'espace intermédiaire situé entre le cathéter (1) et sa partie non repliée vers l'intérieur.

2. Dispositif selon la revendication 1, caractérisé en ce que l'élément tubulaire souple (1) est fermé par un point destiné à la rupture (4) au niveau de l'extrémité (2) située à la côté du patient.

3. Dispositif selon la revendication 1, caractérisé en ce que le cathéter (6) présente à son extrémité éloignée du patient un élément de raccordement pour être relié à la tubulure de raccordement d'une seringue à injections.

4. Dispositif selon la revendication 1, caractérisé en ce que l'extrémité (3), éloignée du patient, de l'élément tubulaire souple (1) est solidement reliée à l'extrémité, située du côté du patient, du cathéter (6).

5. Dispositif selon la revendication 1, caractérisé en ce qu'il est prévu en outre une douille de guidage (14) entourant la partie du cathéter (6) située du côté du patient ainsi que l'élément tubulaire souple (1).

6. Dispositif selon la revendication 1, caractérisé en ce qu'il est dimensionné pour être mis en œuvre en tant que cathéter pour vessie chez les femmes.

7. Dispositif selon la revendication 6, caractérisé en ce que le cathéter (6) mesure environ 16 cm de long et présente un diamètre d'environ 4 mm.

8. Dispositif selon la revendication 6, caractérisé en ce que l'élément tubulaire souple (1) mesure environ 22 cm de long et présente une largeur d'environ 9 mm lorsqu'il est à l'état plat.

9. Dispositif selon la revendication 6, caractérisé en ce que la longueur de la partie, repliée vers l'intérieur, de l'élément tubulaire souple (1) est d'environ 5 à 6 cm.

**Fig. 1**

**Fig. 2**

**Fig. 3**

Fig. 4
Fig. 4a
Fig. 5
Fig. 6

*Fig. 5a*

*Fig. 2a*